(19) Europäisches Patentamt
European Patent Office
Office européen des brevets

(11) Numéro de publication : **0 308 340 B1**

# (12) FASCICULE DE BREVET EUROPEEN

(45) Date de publication du fascicule du brevet : **13.03.91 Bulletin 91/11**

(51) Int. Cl.$^5$ : **C07C 227/18**, C07C 229/24, C07D 209/42

(21) Numéro de dépôt : **88402338.3**

(22) Date de dépôt : **16.09.88**

## (54) Procédé de synthèse d'alpha amino acides N alkyles et de leurs esters. Application à la synthèse de carboxyalkyl dipeptides.

(30) Priorité : **17.09.87 FR 8712901**

(43) Date de publication de la demande : **22.03.89 Bulletin 89/12**

(45) Mention de la délivrance du brevet : **13.03.91 Bulletin 91/11**

(84) Etats contractants désignés : **AT BE CH DE ES FR GB GR IT LI LU NL SE**

(56) Documents cités :
**EP-A- 0 049 605**
**EP-A- 0 049 658**
**EP-A- 0 308 341**
**US-A- 4 296 110**

(56) Documents cités :
**TETRAHEDRON LETTERS, no. 13, 1976, pages 997-1000, Pergamon Press, Oxford, GB; S.-I. YAMADA et al.: "Asymmetric transamination from amino acids (I) Asymmetric synthesis of amino acid by chemical transamination from optically active amino acids to alpha-keto acid"**

(73) Titulaire : **ADIR ET COMPAGNIE**
**22, rue Garnier**
**F-92201 Neuilly sur Seine (FR)**

(72) Inventeur : **Vincent, Michel**
**8 allée du Prunier Hardy**
**F-92220 Bagneux (FR)**
Inventeur : **Baliarda, Jean**
**25 avenue Jeanne d'Arc**
**F-92160 Anthony (FR)**
Inventeur : **Marchand, Bernard**
**71 rue Laveau**
**F-45430 Checy (FR)**
Inventeur : **Remond, Georges**
**9 avenue des Etats-Unis**
**F-78000 Versailles (FR)**

EP 0 308 340 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne un procédé de synthèse industrielle d' α amino diacides N alkylés éventuellement estérifiés et leur application à la synthèse industrielle de carboxy alkyl dipeptides.

Plus spécifiquement, la présente invention concerne un nouveau procédé de synthèse industrielle de dérivés de formule générale (I) :

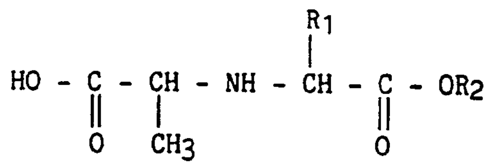

(I)

et leurs sels d'addition à un acide ou base minéral organique, dans laquelle :

$R_1$ est de l'hydrogène ou alcoyle inférieur (de 1 à 6 atomes de carbone) linéaire ou ramifié

$R_2$ est de l'hydrogène ou un groupement alcoyle inférieur (de 1 à 4 atomes de carbone) linéaire ou ramifié.

Les dérivés de formule (I) obtenus selon le procédé de l'invention sont utiles dans la synthèse de carboxy alkyl dipeptides de formule (II) :

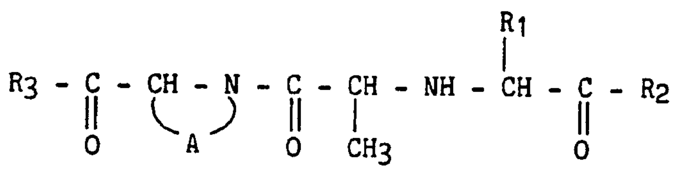

(II)

ainsi que dans celle de leurs sels pharmaceutiquement acceptables, dans laquelle :

– $R_1$ et $R_2$ ont la même signification que dans la formule (I),

– $R_3$ est un atome d'hydrogène ou groupement alcoyle inférieur de 1 à 4 atomes de carbone linéaire ou ramifié,

– La structure

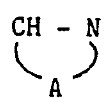

représente l'indoline, l'isoindoline, la tétrahydroquinoléine, la tétrahydroisoquinoléine, le perhydroindole, le perhydroisoindole, la perhydroisoquinoléine, la perhydroquinoléine, le perhydrocyclopenta [b] pyrrole, l'aza-2-bicyclo [2,2,2] octane, l'aza-2 bicyclo [2,2,1] heptane.

Le composé de formule (II) préféré est le perindopril de formule (III)

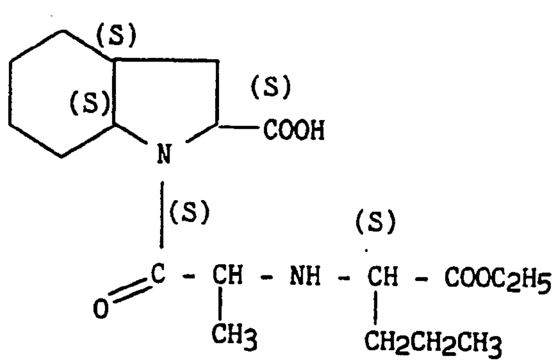

(III)

ou acide {[(éthoxycarbonyl)-1 butylamino-(S)] 2 propionyl-(S)}-1 octahydroindole carboxylique-2 (2S, 3aS, 7aS), ainsi que ses sels d'addition à un acide ou une base pharmaceutiquement acceptables, pour lequel le procédé de la présente invention peut être plus particulièrement appliqué.

Les composés de formule (II) ainsi que leurs sels possèdent des propriétés pharmacologiques intéressantes. Ils exercent notamment une activité inhibitrice sur certaines enzymes, comme les carboxypolypeptidases, les enképhalinases ou la kininase II. Ils inhibent notamment la transformation du décapeptide angiotensine I en l'octapeptide angiotensine II, responsables dans certains cas de l'hypertension artérielle, en agissant sur l'enzyme de conversion.

L'emploi en thérapeutique de ces composés permet donc de réduire ou même supprimer l'activité de ces enzymes responsables de la maladie hypertensive ou de l'insuffisance cardiaque. L'action sur la kininase II a pour résultat l'augmentation de la bradykinine circulante et également la baisse de la tension artérielle par cette voie.

Des composés de formule (II) et, plus particulièrement le composé de formule (III), sa préparation et son utilisation en thérapeutique ont été décrits dans le brevet européen n° 0 049 658.

On peut utiliser les composés de formule (I) pour la préparation des composés de formule (II).

Les composés de formule (I) comprennent deux carbones dits asymétriques pouvant chacun avoir deux configurations R ou S :

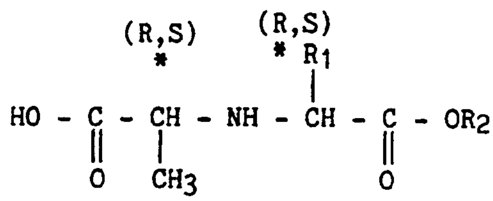

(I)

Les composés de formule (I) existent donc sous forme de quatre stéréoisomères que l'on peut désigner par (R, R) ; (R, S) ; (S, R) ou (S, S) selon la configuration des deux carbones dits asymétriques.

L'art antérieur et notamment le brevet US 4296110 ainsi que la publication Tetrahredon Letters 1976 N° 13 pp 997-1000 fournissent des procédés de synthèse de composés de structure proche ou identique de celle des dérivés de formule (I).

Toutefois cet art antérieur utilise des techniques chromatographiques peu compatibles avec des synthèses de quantités importantes et surtout ne décrit pas de procédé permettant dans de bonnes conditions l'obtention de diastéroisomères spécifiques.

Or, les composés de formule (II) les plus actifs sont ceux pour lesquels les deux carbones de la chaîne latérale ont tous deux la configuration S.

C'est la raison pour laquelle le procédé selon la présente invention s'intéresse plus particulièrement à la synthèse industrielle des composés de formule (I) dans lesquels les deux carbones asymétriques ont tous deux la configuration S.

Peu de procédés de synthèse industrielle spécifiques des dérivés de formule (I) ont été décrits. On connaît la demande de brevet européen n° 0 117 488, très générale, qui utilise des trifluorométhane sulfonates α carboxylés. Toutefois, la stéréochimie des deux produits de départ doit être rigoureusement choisie afin d'obtenir le diastéréoisomère voulu du produit de formule (I).

Il est par ailleurs connu de l'homme de l'Art que, de façon très générale, pour permettre la séparation de diastéréoisomères obtenus au cours de synthèses où la stéréochimie des matières premières n'est pas préalablement fixée, on a recours à des techniques classiques telles que cristallisation fractionnée ou chromatographie sur colonne de silice.

La demanderesse a présentement découvert un procédé de synthèse industrielle des dérivés de formule (I) très intéressant car d'une part particulièrement simple à mettre en uvre, et d'autre part parce qu'il permet, par un choix judicieux des réactifs (catalyseur et solvants) utilisés, l'obtention directe du diastéréoisomère (S, S) avec des rendements industriellement très intéressants.

En outre, le procédé selon l'invention présente l'avantage d'utiliser comme matières premières des dérivés peu coûteux, ce qui, industriellement est d'importance.

Plus particulièrement, le procédé selon la présente invention utilise comme produit de départ un dérivé d'acide aminé naturel, de formule générale (IV) :

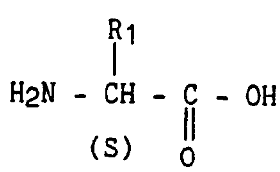

(IV)

dans lequel $R_1$ a la même signification que dans la formule (I),

dans lequel le carbone asymétrique a la configuration S puisqu'il est bien connu de l'homme de l'art que le carbone porteur du carboxyle des amino acides naturels a la configuration S (cystéine exceptée), que l'on traite, lorsque $R_2$ est différent de H en présence de chlorure de thiongle, par un alcool aliphatique inférieur, industriellement disponible à bas prix, de formule $R'_2OH$, $R'_2$ représentant un groupement alcoyle inférieur, comprenant de 1 à 4 atomes de carbone, pour donner un ester de formule (V) :

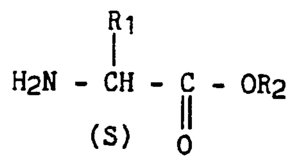

(V)

dans laquelle $R_1$ et $R_2$ ont la même signification que dans la formule (I), lequel est condensé avec l'acide pyruvique, sous hydrogénation en présence de charbon palladié à 5% sous pression et sous léger chauffage, en milieu aqueux,
la pression étant comprise entre 10 et 100 bars, préférentiellement entre 15 et 60 bars,
la température étant comprise entre 10 et 60°C, préférentiellement entre 10 et 40°C,
permettant ainsi l'obtention d'un taux maximal du diastéréoisomère (S, S) du composé de formule (I), avec ledit acide pyruvique $CH3-CO-COOH$, produit naturel, peu coûteux, industriellement disponible, ce seul diastéréoisomère étant finalement obtenu après purification par une cristallisation unique dans un solvant organique polaire, lui-même rigoureusement sélectionné parmi acétonitrile, acétate d'éthyle, alcool aliphatique inférieur, seul ou mélangés à l'eau, ou mélangés entre eux et à l'eau, à condition que le mélange obtenu soit monophasique. L'exemple ci-dessous illustre l'invention, mais ne la limite en aucune façon.

**EXEMPLE : N - [(S) CARBETHOXY - 1 BUTYL] (S) ALANINE**

**STADE A** : Chlorhydrate de L-Norvalinate d'éthyle

Dans un réacteur, placer 35 kg de L-Norvaline dans 300 kg environ d'éthanol dénaturé. Introduire lentement et progressivement 60 kg environ de chlorure de thionyle.

Après un quart d'heure d'agitation, chauffer au reflux pendant 3 heures, puis évaporer l'éthanol sous vide.

Reprendre le résidu par 300 litres de cyclohexane, et porter à ébullition. Après refroidissement, filtrer, laver au cyclohexane et sécher. On obtient 52,9 kg de chlorhydrate de L - Norvalinate d'éthyle, soit un rendement de 97,6%.

Le produit ainsi obtenu est utilisé tel quel au stade suivant.

**STADE B** : N-[(S) carbéthoxy-1 butyl] (S) alanine

Dans une cuve équipée d'un agitateur, placer 45 kg de chlorhydrate de L-Norvalinate d'éthyle obtenu au stade précédent dans environ 110 litres d'eau.

Alcaliniser et verser ensuite très progressivement 23 kg d'acide pyruvique dans la solution précédemment obtenue et agiter le milieu réactionnel pendant 30 minutes.

Placer dans un appareil à hydrogéner du charbon palladié à 5% en suspension dans l'eau, et la solution alcaline de L-Norvalinate d'éthyle précédemment obtenue.

Hydrogéner sous pression (30 bars) à température ambiante pendant une journée environ.

Filtrer sous vide, et évaporer le filtrat sous pression réduite, essorer et sécher. Traiter le résidu obtenu par de l'éthanol ; éliminer l'insoluble, constitué de chlorure de sodium, par filtration et le rincer à l'éthanol. Réunir les solutions éthanoliques ; évaporer l'éthanol sous pression réduite et, cristalliser le résidu dans l'acétonitrile

On obtient 34,3 kg de N-[(S) carbéthoxy-1 butyl] (S) alanine, soit un rendement de 63,9%.

## Revendications

1. Procédé de synthèse industrielle de composés de formule (I)
dans laquelle :

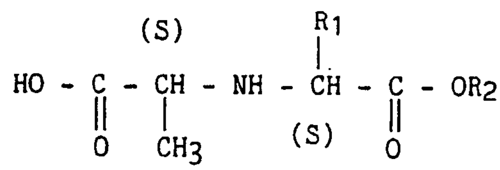

(I)

$R_1$ est alcoyle inférieur (de 1 à 6 atomes de carbone) linéaire ou ramifié

$R_2$ est de l'hydrogène ou un groupement alcoyle inférieur (de 1 à 4 atomes de carbone) linéaire ou ramifié,

les deux atomes de carbone asymétrique ayant tous deux la configuration S,

caractérisé en ce que l'on utilise comme matière première un dérivé de formule (IV) :

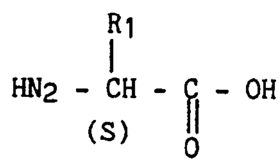

(IV)

dans laquelle :

– $R_1$ a la même signification que dans la formule (I), le carbone asymétrique ayant la configuration S, qui lorsque $R_2$ est différent de l'atome d'hydrogène, est estérifié, en présence de chlorure de thionyle, par un alcool aliphatique inférieur de formule générale $R'_2OH$ dans laquelle $R'_2$ représente un groupement alcoyle inférieur linéaire ou ramifié de 1 à 4 atomes de carbone,

pour obtenir un dérivé de formule (V) :

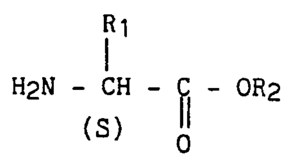

(V)

dans laquelle :

– $R_1$ et $R_2$ ont la même signification que dans la formule (I), le carbone asymétrique ayant la configuration S,

lequel est condensé avec l'acide pyruvique sous hydrogénation en présence de charbon palladié à 5% sous pression et sous léger chauffage,

la pression étant comprise entre 10 et 100 bars,

la température étant comprise entre 10 et 60°C,

en milieu aqueux,

pour permettre l'obtention d'un taux optimal du diastéreoisomère du derivé de formule (I) dans lequel les deux carbones asymétriques ont la configuration S,

ce seul diastéréoisomère étant finalement obtenu après purification par une cristallisation unique dans un solvant organique polaire, lui-même rigoureusement sélectionné parmi acétonitrile, acétate d'éthyle, alcool aliphatique inférieur, seul ou mélangés à l'eau, ou mélangés entre eux et à l'eau, à condition que le mélange obtenu soit monophasique.

2. Procédé de synthèse industrielle selon la revendication 1, permettant l'obtention du dérivé de formule (I) dans lequel :

$R_1$ est un groupement n propyle,

$R_2$ est un groupement éthyle.

3. Procédé selon l'une quelconque des revendications 1 et 2, caractérisé en ce que la pression retenue pour l'hydrogénation catalytique soit comprise entre 15 et 60 bars.

4. Procédé selon l'une quelconque des revendications 1 à 3, caractérisé en ce que la température retenue pour l'hydrogénation catalytique soit comprise entre 10 et 40°C.

5. Procédé selon l'une quelconque des revendications 1 à 4, de synthèse industrielle du composé de formule (I) appelé dérivé de formule (Ia), dans lequel :

$R_1$ est un groupement n propyle,

$R_2$ est un groupement éthyle,

caractérisé en ce que, en fin de synthèse, le produit de formule (I) est purifié par une cristallisation unique dans l'acétonitrile.

6. Utilisation d'un dérivé de formule (I) ou (Ia) obtenu selon l'une quelconque des revendications 1 à 5 pour la synthèse de dérivés de formule (II) :

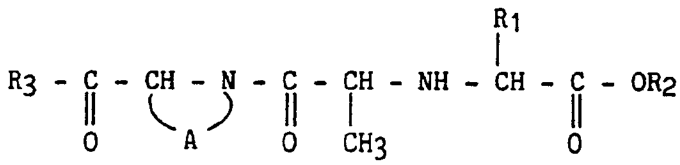 (II)

et leurs sels pharmaceutiquement acceptables, dans laquelle :

– $R_1$ et $R_2$ ont la même signification que dans la formule (I),
– $R_3$ est un atome d'hydrogène ou un groupement alcoyle de 1 a 4 atomes de carbone linéaire ou ramifié,
– La structure

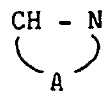

représente l'indoline, l'isoindoline, la tétrahydroquinoléine, la tétrahydroisoquinoléine, le perhydroindole, le perhydroisoindole, la perhydroisoquinoléine, la perhydroquinoléine,

le perhydrocyclopenta [b] pyrrole, l'aza-2-bicyclo[2,2,2]octane, l'aza-2 bicyclo[2,2,1]heptane.

7. Utilisation selon la revendication 6, du dérivé de formule (Ia) obtenu selon la revendication 5 dans la synthèse de dérivés de formule (II).

8. Utilisation selon l'une des revendications 6 et 7, du dérivé de formule (Ia) dans la synthèse de dérivés de formule (II) dans laquelle la structure

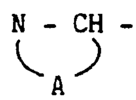

représente le perthydroindole

9. Utilisation selon les revendications 7 à 8 du dérivé de formule (Ia) dans la synthèse de l'acide {[(éthoxycarbonyl)-1 butylamino-(S)]-2 propionyl-(S)}-1 octahydroindole carboxylique-2 (2S, 3aS, 7aS) ou perindopril.

## Ansprüche

1. Verfahren zur technischen Herstellung von Verbindungen der Formel (I)

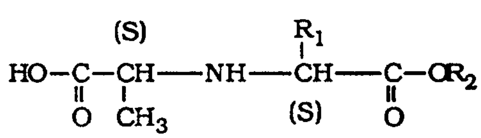 (I)

in der

$R_1$ eine geradkettige oder verzweigte niedrigmolekulare Alkylgruppe (mit 1 bis 6 Kohlenstoffatomen) und

$R_2$ ein Wasserstoffatom oder eine geradkettige oder verzweigte niedrigmolekulare Alkylgruppe (mit 1 bis 4 Kohlenstoffatomen) bedeuten,

wobei die beiden asymmetrischen Kohlenstoffatome stets in der SKonfiguration vorliegen,

**dadurch gekennzeichnet**, daß man als Ausgangsmatenal ein Derivat der Formel (IV)

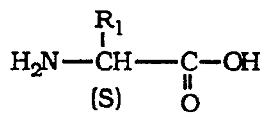 (IV)

verwendet, in der $R_1$ die bezüglich der Formel (I) angegebenen Bedeutungen besitzt, und das asymmetrische Kohlenstoffatom in der S-Konfiguration vorliegt,
welches dann, wenn $R_2$ von dem Wasserstoffatom verschieden ist, in Gegenwart von Thionylchlorid mit einem niedrigmolekularen aliphatischen Alkohol der allgemeinen Formel $R'_2OH$, worin $R'_2$ für eine geradkettige oder verzweigte niedrigmolekulare Alkylgruppe mit 1 bis 4 Kohlenstoffatomen steht, verestert wird zur Bildung eines Derivats der Formel (V)

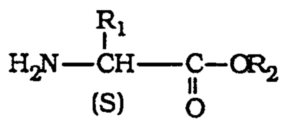 (V)

in der
$R_1$ und $R_2$ die bezüglich der Formel (I) angegebenen Bedeutungen besitzen, wobei das asymmetrische Kohlenstoffatom in der S-Konfiguration vorliegt, welches Derivat unter Hydrieren in Gegenwart von 5% Palladium enthaltender Kohle in einem wässrigen Medium unter Druck und unter schwachem Erwärmen mit Brenztraubensäure kondensiert wird, wobei der Druck zwichen 10 und 100 bar und die Temperatur zwischen 10 und 60°C liegen, zur Erzeugung einer optimalen Ausbeute des Diastereoisomeren des Derivats der Formel (I), in der die beiden asymmetrischen Kohlenstoffatome in der S-Konfiguration vorliegen,
wobei dieses einzige Diastereoisomere schließlich erhalten wird nach einer Reinigung durch eine einmalige Kristallisation aus einem polaren organischen Lösungsmittel, welches ausgewählt ist aus Acetonitril, Ethylacetat und niedrigmolekularen aliphatischen Alkoholen, welche allein oder in Form von Mischungen mit Wasser oder in Form von Mischungen miteinander und mit Wasser, mit der Maßgabe, daß die erhaltene Mischung einphasig ist, eingesetzt werden können.

2. Verfahren zur technischen Synthese gema Anspruch 1, zur Bildung eines Derivats der Formel (I), in der
$R_1$ eine n-Propylgruppe und
$R_2$ eine Ethylgruppe bedeuten.

3. Verfahren nach einem der Ansprüche 1 und 2, **dadurch gekennzeichnet**, daß der bei der katalytischen Hydrierung angewandte Druck zwischen 15 und 60 bar gehalten wird.

4. Verfahren nach einem der Ansprüche 1 bis 3, **dadurch gekennzeichnet**, daß die bei der katalytischen Hydrierung angewandte Temperatur zwischen 10 und 40°C gehalten wird.

5. Verfahren nach einem der Ansprüche 1 bis 4 zur technischen Synthese der Verbindung der Formel (I), die auch als Derivat der Formel (Ia) bezeichnet wird,in der $R_1$ eine n-Propylgruppe und $R_2$ eine Ethylgruppe bedeuten, **dadurch gekennzeichnet**, daß das Produkt der Formel (I) nach der Synthese durch eine einmalige Kristallisation aus Acetonitril gereinigt wird.

6. Verwendung eines Derivats der Formel (I) oder (Ia), erhalten nach einem der Ansprüche 1 bis 5 für die Herstellung von Derivaten der Formel (II)

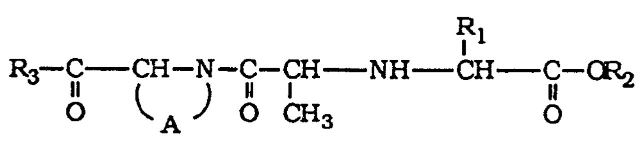 (II)

und deren pharmazeutisch annehmbaren Salzen, worin
$R_1$ und $R_2$ die bezüglich der Formel (I) angegebenen Bedeutungen besitzen,
$R_3$ ein Wasserstoffatom oder eine geradkettige oder verzweigte Alkylgruppe mit 1 bis 4 Kohlenstoffatomen bedeutet,
die Gruppe

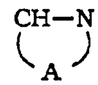

für Indolin, Isoindolin, Tetrahydrochinolin,

Tetrahydroisochinolin, Perhydroindol, Perhydroisoindol, Perhydrosisochinolin, Perhydrochinolin, Perhydrocyclopenta[b]pyrrol, 2-Aza-bicyclo[2.2.2]octan oder 2-Aza-bicyclo[2.2.1]heptan steht.

7. Verwendung nach Anspruch 6 des Derivats der Formel (Ia), erhalten gemäß Anspruch 5 für die Herstellung von Derivaten der Formel (II).

8. Verwendung nach einem derAnsprüche 6 und 7 des Derivats der Formel (Ia) für die Herstellung von Derivaten der Formel (II), in der die Gruppe

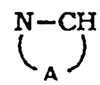

für Perhydroindol steht.

9. Verwendung nach den Ansprüchen 7 bis 8 des Derivats der Formel (Ia), für die Herstellung von 1-{2-(1-(Ethoxycarbonyl)-(S)-butylamino]-(S)-propionyl}-octahydroindol-2(2S, 3aS, 7aS)-carbonsäure oder Perindopril.

## Claims

1. Process for the industrial synthesis of compounds of formula (I) :

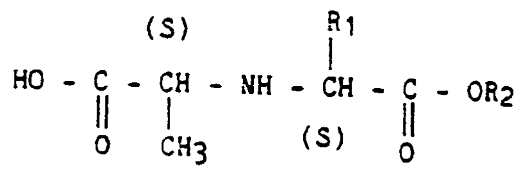

(I)

in which :

$R_1$ is linear or branched lower alkyl (having from 1 to 6 carbon atoms)

$R_2$ is hydrogen or a linear or branched lower alkyl group (having from 1 to 4 carbon atoms),

the two asymmetric carbon atoms both having the S configuration, characterised in that the starting material employed is a derivative of formula (IV) :

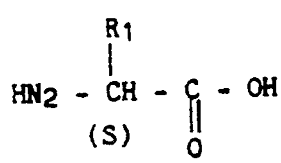

(IV)

in which :

– $R_1$ has the same meaning as in formula (I), the asymmetric carbon atom having the S configuration, which, when $R_2$ is other than the hydrogen atom, is esterified in the presence of thionyl chloride with a lower aliphatic alcohol of the general formula $R'_2OH$ in which $R'_2$ denotes a linear or branched lower alkyl group having from 1 to 4 carbon atoms,

to obtain a derivative of formula (V) :

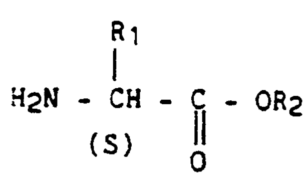

(V)

in which :

– $R_1$ and $R_2$ have the same meaning as in formula (I), the asymmetric carbon atom having the S configuration,

which is condensed with pyruvic acid with hydrogenation in the presence of 5% palladium/carbon under pressure and with gentle heating,

the pressure being between 10 and 100 bars,

the temperature being between 10 and 60°C,

in an aqueous medium,

to make it possible to obtain an optimum proportion of the diastereoisomer of the derivative of formula (I) in which the two asymmetric carbons have the S configuration, this diastereoisomer alone finally being obtained after purification by a single crystallisation from a polar organic solvent, itself strictly chosen from acetonitrile, ethyl acetate and a lower aliphatic alcohol, by itself or mixed with water, or mixed with each other and with water, provided that the mixture obtained is a single phase.

2. Industrial synthesis process according to claim 1, making it possible to obtain the derivative of formula (I) in which :

$R_1$ is an n-propyl group,

$R_2$ is an ethyl group.

3. Process according to either of claims 1 and 2, characterised in that the pressure established for the catalytic hydrogenation is between 15 and 60 bars.

4. Process according to any one of claims 1 to 3, characterised in that the temperature established for the catalytic hydrogenation is between 10 and 40°C.

5. Process according to any one of claims 1 to 4, for the industrial synthesis of the compound of formula (I) referred to as derivative of formula (Ia), in which :

$R_1$ is an n-propyl group,

$R_2$ is an ethyl group,

characterised in that, when the synthesis is finished, the product of formula (I) is purified by a single crystallisation from acetonitrile.

6. The use of a derivative of formula (I) or (Ia) obtained according to any one of claims 1 to 5 for the synthesis of derivatives of formula (II) :

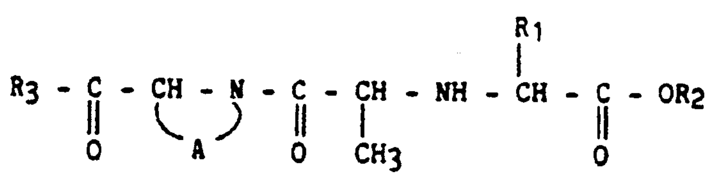

(II)

and their pharmaceutically acceptable salts, in which :

– $R_1$ and $R_2$ have the same meaning as in formula (I),

– $R_3$ is a hydrogen atom or a linear or branched alkyl group having from 1 to 4 carbon atoms,

– the structure

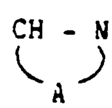

denotes indoline, isoindoline, tetrahydroquinoline, tetrahydroisoquinoline, perhydroindole, perhydroisoindole, perhydroisoquinoline, perhydroquinoline, perhydrocyclopenta[b]pyrrole, 2-azabicyclo[2,2,2]octane or 2-azabicyclo[2,2,1]heptane.

7. Use according to claim 6 of the derivative of formula (Ia) obtained according to claim 5 in the synthesis of derivatives of formula (II).

8. Use according to either of claims 6 and 7 of the derivative of formula (Ia) in the synthesis of derivatives of formula (II) in which the structure

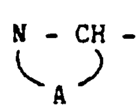

denotes perhydroindole.

9. Use according to claims 7 to 8 of the derivative of formula (Ia) in the synthesis of (2S, 3aS, 7aS)-1-{2-[1-(ethoxycarbonyl)-(S)-butylamino]-(S)-propionyl}-octahydroindole-2-carboxylic acid or perindopril.